# EUROPÄISCHE PATENTSCHRIFT

(11) **EP 2 367 577 B1**
(45) Veröffentlichungstag und Bekanntmachung des Hinweises auf die Patenterteilung: **04.06.2014**
(21) Anmeldenummer: 09796958.8
(22) Anmeldetag: 15.12.2009
(51) Int. Cl.: G09B 23/30

(54) **MESSAPPARATUR FÜR UNTERDRUCKTHERAPIESYSTEME FÜR DIE WUNDBEHANDLUNG**
MEASUREMENT APPARATUS FOR VACUUM THERAPY SYSTEMS FOR WOUND TREATMENT
APPAREIL DE MESURE POUR DES SYSTÈMES DE TRAITEMENT DES PLAIES PAR PRESSION NÉGATIVE

(30) Priorität: 22.12.2008 DE 102008064510
(43) Veröffentlichungstag der Anmeldung: 28.09.2011
(73) Patentinhaber: Paul Hartmann Aktiengesellschaft, 89522 Heidenheim (DE)
(72) Erfinder: ECKSTEIN, Axel, 89522 Heidenheim (DE); JUNGINGER, Martin, 89568 Hermaringen (DE)
(74) Vertreter: DREISS Patentanwälte PartG mbB
(86) Internationale Anmeldenummer: PCT/EP2009/008959
(87) Internationale Veröffentlichungsnummer: WO 2010/072349

(56) Entgegenhaltungen:
- WO-A1-2005/105175
- WO-A1-2005/105179
- WO-A2-2009/059444
- US-A1- 2008 077 091

## Beschreibung

Die Erfindung betrifft eine Messapparatur für Unterdrucktherapiesysteme für die Wundbehandlung umfassend eine künstliche Wundeinheit, die eine Wandung umfasst, die eine wenigstens einseitig offene Wundkavität einschließt, wobei in der Wandung Fluiddurchlässe vorgesehen sind, die über Fluidzuleitungen mit einem Fluid versorgbar sind und wobei die offene Seite der Wundkavität mit einem Unterdrucktherapiesystem abdeckbar ist, wobei über das Unterdrucktherapiesystem an der Wundkavität über eine Unterdruckerzeugungseinrichtung der Messapparatur ein Unterdruck anlegbar ist.

Eine entsprechende Einrichtung ist aus der US 2008/0077091 A1 bekannt, die ein System zum Testen von Unterdruckwundauflagen beschreibt. Hierzu ist eine simulierte Wunde mit einem Gehäuse und einer Wundkavität innerhalb des Gehäuses vorgesehen. Über die Form und die Größe der Kavität wird ein bestimmter Wundtyp repräsentiert. Über dieser Wundkavität kann ein Wundverband positioniert werden und es wird eine Unterdruckeinrichtung an die Wundkavität angelegt. Darüber hinaus soll wenigstens ein Sensor vorgesehen sein, um wenigstens einen Parameter innerhalb der simulierten Wunde aufzunehmen. Des Weiteren wird die Vorsehung einer Fluidquelle zur Bereitstellung von Fluiden in der wundkavität, die der Simulation von Wundexsudat dienen soll, offenbart. Die erfassten Daten können dann mittels eines Computers ausgewertet werden, um so ein Indikator dafür zu sein, wie ein jeweiliges System funktioniert. Schließlich offenbart die US 2008/0077091 A1 ein Leckagemodell. Osnabrück, 2006 ein Testsystem bekannt, das der Überprüfung von Wundauflagen unter Unterdruckbedingungen dient, wobei die Messanordnung die Verwendung eines Rinderherzens oder einer realen Wunde bedarf.
Weitere Messapparaturen sind beispielsweise aus der DE 102 26 532 B3 vorbekannt, die eine Messapparatur zur Ermittlung von Druckwerten bei Pflasteranwendungen beschreibt. Dabei ist ein Narbenmodell vorgesehen, wobei die Messapparatur zur Ermittlung von Druckwerten bei Narbenreduktionspflastern dient. Eine entsprechende Messapparatur für Narbenreduktionspflaster eignet sich jedoch nicht für die Überprüfung von Wundauflagen, wie sie bei nässenden Wunden, bei denen Wundexsudat entsteht, Verwendung finden kann.

Eine weitere Testapparatur ist aus der GB 2 362 466 A vorbekannt, die jedoch ebenfalls keine Einrichtung zum Testen von Unterdrucktherapiesystemen in der Wundbehandlung offenbart, sondern ein System, bei dem eine Wundauflage in eine künstliche Wunde eingebracht wird und künstliche Wundflüssigkeit in die Wunde eingeleitet und über weitere Ausgänge wieder aus der Wunde entfernt wird, wobei auf diese Weise beispielsweise die chemische Zusammensetzung der Wundflüssigkeit ermittelt werden kann.

Nachteilig bei den genannten Messapparaturen für Unterdrucktherapiesysteme ist dabei, dass die Wundumgebung nur mangelhaft abgebildet werden kann. Es ist daher Aufgabe der Erfindung, eine Messapparatur für Unterdrucktherapiesysteme für die Wundbehandlung bereitzustellen, mit der die natürliche Wundumgebung besser abgebildet werden kann.

Die Erfindung löst diese Aufgabe durch eine Messapparatur mit den Merkmalen des Anspruchs 1, bei der eine regelbare Heizeinrichtung zur Temperierung der Wundeinheit und/oder des Fluids vorgesehen ist und die Wandung der künstlichen Wunde durch ein eine offene Porosität aufweisendes Material gebildet ist.

Durch die Verwendung einer regelbaren Heizeinrichtung kann die zu testende Wundauflage über die Temperaturregelung der künstlichen Wunde sowie das der künstlichen Wunde zugeführte Wundfluid realitätsnäher getestet werden, da sich eine beheizte Wunde und ein vorgewärmtes künstliches Wundexsudat näher an den tatsächlichen Gegebenheiten einer Wunde befinden als eine Durchführung von Tests bei Raumtemperatur. Insbesondere lassen sich realitätsnahe Wundsituationen bei einer der Körpertemperatur angenäherten Temperatur durchführen.

Im Gegensatz zu einer reinen Temperaturerfassung bzw. zu einer Durchführung der Versuche in einem temperierten Raum bietet die regelbare Beheizung von Wundeinheit und/oder Fluid den Vorteil, dass hierdurch dem Phänomen entgegengewirkt werden kann, dass durch die Überführung von Wundexsudat in die Gasphase eine Verdampfungsenthalpie benötigt wird. Dieser für die Verdampfung benötigten Energiemenge, die zu einer Abkühlung des Fluids und der Umgebung führt, die ansonsten die Testbedingungen beeinflusst, kann durch die Verwendung einer regelbaren Heizeinrichtung entgegengewirkt werden.

Darüber hinaus ist die Viskosität und die Verdunstungsrate von Flüssigkeit abhängig von der Temperatur. Die Untersuchung und auch die Regulierung dieser Parameter kann über die regelbare Heizeinrichtung variiert werden.

Hierdurch lassen sich besonders realitätsnahe Wundsituationen simulieren.

Besonders bevorzugt ist ein regelbares Heiz- bzw. Wasserbad zur Temperierung der künstlichen Wunde und des Fluids, das als künstliches Wundexsudat dient.

Dabei bietet die Verwendung von Unterdrucktherapiesystemen zur Wundbehandlung eine Reihe von Vorteilen. Durch die mechanischen Kräfte, die auf die Zellen einwirken, erfolgt die Wundheilung in der Art einer Kompressionstherapie. Der Begriffe Unterdrucktherapiesystem umfasst dabei eine Wundauflage zum Ein- oder Auflegen auf die Wunde, eine Abdeckschicht, die gasdicht ist und die Wunde abdichtet sowie einen Drainageschlauch zum Anlegen des Unterdrucks sowie zur Ableitung von Wundexsudat und ggf. von zugeführter Spülflüssigkeit.

Darüber hinaus kann durch die kontinuierliche Ableitung von Wundsekret über den anliegenden Unterdruck eine positive Auswirkung erzielt werden und es kann darüber hinaus die Keimbesiedlung der Wunde verringert werden.

Dabei kann es besonders bevorzugt sein, dass die künstliche Wundeinheit an der Messapparatur lösbar und auswechselbar festlegbar ist. Hierzu kann die Messapparatur eine Aufnahme für die künstliche Wunde aufweisen, wobei die Aufnahme z. B. aus einer Wanne, die durch eine die künstliche Wunde aufnehmende Platte abgedeckt ist, gebildet sein kann. In der Wanne kann dann besonders bevorzugt das Heizbad angeordnet sein.

Insbesondere können so Wunden verschiedener Größe, aber auch verschiedene Geometrien, wie insbesondere tunnelnde Wunden oder große flache Wunden bzw. tiefe Wunden simuliert werden. Die künstlichen Wundeinheiten können dabei sämtlich mit der vorhandenen Messapparatur verbunden werden und insbesondere in die Aufnahme eingesetzt werden, so dass durch die Verwendung einer Messapparatur mit verschiedenen Wundeinheiten Kosten eingespart werden können. Darüber hinaus kann das Verhalten von Unterdrucktherapiesystemen auf unterschiedlichen Wundgeometrien und/oder Wundbeschaffenheiten, wie beispielsweise flächige Wunden im Gegensatz zu kavitätischen Wunden, getestet werden. Auch ist ein Vergleich zwischen einem weichen Untergrund, wie er einen Gewebeschaden simulieren kann, bzw. einem harten Untergrund, der einen Gewebeschaden bis auf die Knochen simulieren kann, bei konstant gehaltenen weiteren Prüfparametern möglich.

Besonders bevorzugt ist dabei, dass ein Kraftsensor in der Wundkavität vorgesehen ist. Der Kraftsensor kann dabei in der künstlichen Wunde festgelegt werden. Mit diesem Kraftsensor kann die Kraft gemessen werden, die von dem zur Untersuchung stehenden Unterdrucktherapiesystem auf eine künstliche Wunde ausgeübt wird. Die Parameter, die diese Kraft beeinflussen, sind zum einen die Deformierbarkeit der Wunde sowie die Beschaffenheit der Wunde, sowie die Verformbarkeit der Wundauflage sowie ggf. der Abdeckschicht des Unterdrucktherapiesystems. Über die ermittelten Kraftwerte kann eine Aussage über die tatsächlich eingestellten Druckverhältnisse in der Wundkavität und damit auf eine Wunde gemacht werden. Dieser tatsächlich eingestellte Druck, der über die gemessene Kraft ermittelt werden kann, ist verschieden von dem eingestellten Unterdruck (Luftdruck), der über das Unterdrucktherapiesystem an die Wunde angelegt wird, und darüber hinaus ebenfalls verschieden vom eingestellten Unterdruck innerhalb der Wundkavität.

Darüber hinaus ist die Wandung der künstlichen Wunde durch ein eine offene Porosität aufweisendes Material gebildet. Insbesondere kann ein Glasmaterial bzw. ein Glaskörper eingesetzt werden, der eine offene Porosität aufweist. Solche Glasfritten entstehen bei der Herstellung von Glasschmelzen.

Durch die Verwendung eines eine offene Porosität aufweisenden Materials wird der Vorteil erreicht, dass hierdurch die Fluidverhältnisse in einer Wunde besonders gut simuliert werden können. Auf diese Weise kann das Fluid, das über die Fluidzuleitungen zur Wandung geleitet wird über die Poren der künstlichen Wunde, die die Fluiddurchlässe bilden, durch Kapillarwirkung besonders homogen über die gesamte Wandungsfläche verteilt und abgegeben werden. Eine homogene Flüssigkeitsabgabe entspricht dabei den natürlichen Wundverhältnissen.

Besonders bevorzugt kann die künstliche Wunde gegebenenfalls in ihrer Aufnahme an einer Halterung festgelegt und an der Halterung verschwenkbar gelagert sein, wobei die Verschwenkung in verschiedenen Positionen arretierbar oder festlegbar ist. Dabei kann eine Arretierung oder Festlegung in jeder beliebigen Position oder lediglich in vorgegebenen diskreten Positionen möglich sein. Durch die Verschwenkbarkeit der künstlichen Wunde wird erreicht, dass die Absorptionseigenschaften und Abzugsraten von Flüssigkeiten über ein Unterdrucktherapiesystem besonders gut ermittelt werden können, weil die Fluidverteilung innerhalb des Unterdrucktherapiesystems hierüber variiert werden kann.

Darüber hinaus kann durch die Verschwenkbarkeit eine Wunde dahingehend verbessert nachgestellt werden, indem beispielsweise bei Behandlung einer Wunde am Bein bei einem stationär behandelten Patienten sich die Wunde in horizontaler Lage befindet, wohingegen bei einem ambulant behandelten Patienten sich die Wunde in weiten Teilen des Tages in vertikaler Lage befindet. Hierdurch werden die Flüssigkeitsverteilungen in der Wundkavität beeinflusst und es können somit bestimmte Effekte an dem Unterdrucktherapiesystem überprüft werden.

Besonders bevorzugt ist die Vorsehung einer Spüllösungszuleitung, die mit der Wundkavität verbunden ist. Über diese Spüllösungszuleitung kann eine Spüllösung, beispielsweise eine Salz- oder Ringerlösung, zugeführt werden. Hierdurch kann eine zusätzliche Therapiemaßnahme neben der Anwendung eines Unterdrucks getestet werden. Es können auch Systeme getestet werden, die über eine kombinierte Saug-/Spülvorrichtung verfügen. Der Einsatz einer Spüllösung kann darüber hinaus auch ohne Verwendung einer regelbaren Heizeinrichtung vorgesehen werden.

Des weiteren kann durch eine spezielle Halterung der Einfluss der Gravitation auf die Unterdrucktherapie beobachtet werden. Insbesondere ist hierbei eine verschwenkbar gelagerte künstliche Wunde vorgesehen, die in verschiedenen Positionen festlegbar ist.

Die Erfindung wird im Folgenden anhand einer Zeichnung näher erläutert. Dabei zeigen:
- Figur 1: eine perspektivische Darstellung der erfindungsgemäßen Messapparatur;
- Figur 2: einen Schnitt durch eine erfindungsgemäße Messapparatur;
- Figur 3a: eine Ansicht von oben der Messapparatur mit Unterdrucktherapiesystem;
- Figur 3b: einen Schnitt durch eine Messapparatur gemäß Figur 3a und
- Figur 4: eine Messapparatur im Betrieb mit angeschlossener Unterdruckeinheit.

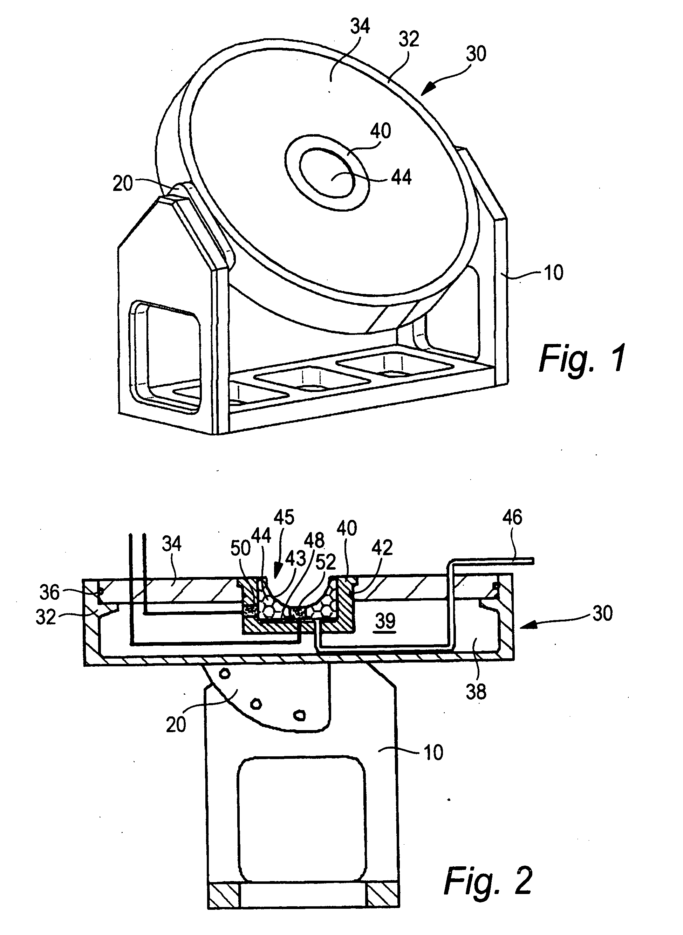

Figur 1 zeigt eine Messapparatur mit einer Halterung 10, die als Aluminiumgestell ausgebildet ist. Des Weiteren umfasst die Messapparatur eine künstliche Wundeinheit 44, die in einer Aufnahme 30, die eine Aluminiumwanne 32 umfasst, die mit einer Edelstahlplatte 34 abgedeckt ist, aufgenommen ist. Hierdurch wird ein Hohlraum 38 unterhalb der Edelstahlplatte 34 und innerhalb der Wanne 32 ausgebildet. Die Edelstahlplatte 34 kann dabei vorzugsweise plan ausgebildet sein.

Zwischen Edelstahlplatte 34 und der Aluminiumwanne 32 kann eine Dichtung 36 eingebracht werden, so dass kein Fluid, das im Hohlraum 38 vorgesehen sein kann, im Bereich zwischen Aluminiumwanne 32 und Edelstahlplatte 34 austreten kann.

Des Weiteren ist eine künstliche Wundeinheit, die durch eine Glasfritte gebildet wird und mit dem Bezugszeichen 44 versehen ist, mittig in der runden Edelstahlplatte 34 angeordnet und dort in einem Gehäuse 40 aufgenommen. Die künstliche Wundeinheit 44 ist dabei gegenüber der Edelstahlplatte 34 durch eine Dichtung 42 abgedichtet, die zwischen Gehäuse 40 und der Platte 34 angeordnet ist.

Die Wundeinheit ist an der Halterung 10 verschwenkbar gelagert, wobei hierzu eine Arretierplatte 20 vorgesehen ist, die eine Arretierung der Wundeinheit 44 in verschiedenen diskreten Schwenkpositionen um eine nicht dargestellte Schwenkachse ermöglicht. Die Wundeinheit 44 kann dann in der voreingestellten Lage festgelegt werden, um eine dauerhafte Überprüfung einer Wundsimulation in einer bestimmten Lage zu erreichen.

Figur 2 zeigt nun die Apparatur gemäß Figur 1 im Schnitt, wobei die künstliche Wunde 44 über eine Fluidzuleitung 46 mit Fluid versorgt werden kann, und die Fluidzuleitung 46 mit einem Fluidreservoir, das nicht dargestellt ist, verbunden ist. Über die Fluidzuleitungen 46 wird Fluid, insbesondere künstlich hergestelltes und dem natürlichen Wundexsudat nachempfundenes Fluid, der künstlichen Wunde zugeleitet, wobei unterhalb der künstlichen Wunde 44 ein Flüssigkeitsverteilraum 48 zur gleichmäßigen Verteilung des Fluids über die gesamte Fläche der künstlichen Wunde 44 bereitsteht.

Da es sich bei der künstlichen Wunde 44 um eine Glasfritte handelt, die eine offene Porosität aufweist, existieren nahezu unendlich viele Fluiddurchlässe, über die Fluid aus der Wandung 43 in die künstliche Wundkavität 45 einzutreten vermag.

Darüber hinaus sind Sensoren vorgesehen, wobei ein Kraftsensor 52 in der Glasfritte angeordnet ist, um so die tatsächlich auf den Wundgrund ausgeübte Kraft bestimmen zu können, und darüber hinaus ist ein Drucksensor 50 vorgesehen, der außerhalb der künstlichen Wunde 44 vorgesehen ist und im Gehäuse 40 gehalten ist und den tatsächlichen Druck innerhalb der künstlichen Wunde aufzunehmen vermag.

Im Hohlraum 38 ist darüber hinaus eine regelbare Heizeinrichtung vorgesehen, wobei die Heizeinrichtung hier als regelbares Heizbad realisiert ist. Die Temperatur des Heiz- oder Wasserbades 39 ist dabei thermoregulierbar, so dass die künstliche Wunde 44, aber auch das Fluid, das über die Fluidzuleitung 46 zur künstlichen Wunde 44 geleitet wird, durch das temperierte und regulierbare Wasserbad hindurchgeleitet werden und so auf einer konstanten Temperatur gehalten sind. Auf diese Weise können Effekte, die ansonsten durch Verdunstungskälte in der Wundkavität 45 entstehen, ausgeglichen werden.

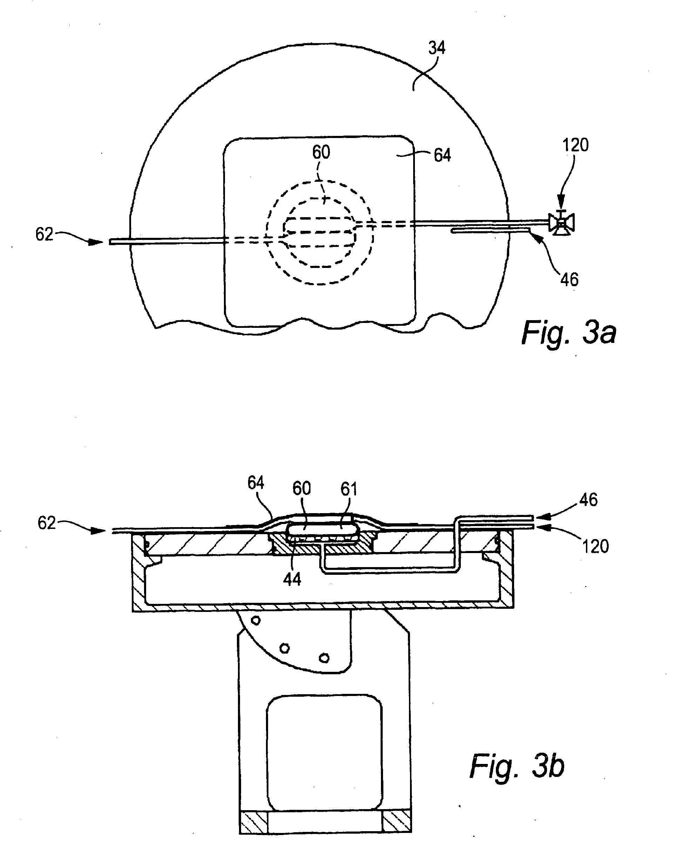

Die Figuren 3 zeigen nun eine entsprechende Ausgestaltung mit einer aufgebrachten Wundauflage als Unterdrucktherapiesystem für die Wundbehandlung. Hierbei ist im Vergleich zu Figur 2 eine andere Wundeinheit 44 vorgesehen, die nun eine flache Wunde nachbildet. Das Unterdrucktherapiesystem für die Wundbehandlung ist hier mit dem Bezugszeichen 60 bezeichnet. Es umfasst eine Wundauflage 61 sowie eine Abdeckung 64 und einen Drainageschlauch 62. Oberhalb der Wundauflage 61 wird ein Unterdruck über einen Drainageschlauch 62 an das Unterdrucktherapiesystem 60 angelegt. Über der Wundauflage 61 ist des Weiteren eine Abdeckung 64 angebracht, die als Polyurethanfilm ausgebildet ist, und die den Drainageschlauch 62 möglichst leckagesicher mit der Wundauflage 61 verbindet und gegenüber der Umgebung abdichtet. Die Abdeckung 64 ist dabei deutlich größer gestaltet als die Wundauflage 61 und überragt dieses allseitig und wird an der Edelstahlplatte 34 klebend festgelegt. Wird nun ein Unterdruck über den Drainageschlauch 62 an die Wundauflage 61 angelegt, so erfolgt eine Beaufschlagung der künstlichen Wunde 44 mit ihrer Wundkavität 45 mit dem entsprechenden verringerten Druck, wobei über in Figur 3 nicht dargestellte Sensoren sowohl die Druckverhältnisse als auch die Kraftverhältnisse in der künstlichen Wunde 44 nachgestellt und gemessen werden können.

Darüber hinaus zeigen die Figuren 3 a) und b) eine Spüllösungszuleitung 120 zum Einleiten einer Spüllösung, z.B. Kochsalz- oder Ringerlösung in die Wundkavität 45. Die zugeführte Spüllösung kann dann über die Drainageleitung 62 wieder aus der Wunde 44 abgezogen werden.

Bei der Wundauflage 61 kann es sich insbesondere um eine absorbierende Wundauflage handelt, wobei Schäume, aber auch Gewebe und Vliese eingesetzt sein können. Bei der Abdeckung 64handelt es sich um eine gasdichte Folie.

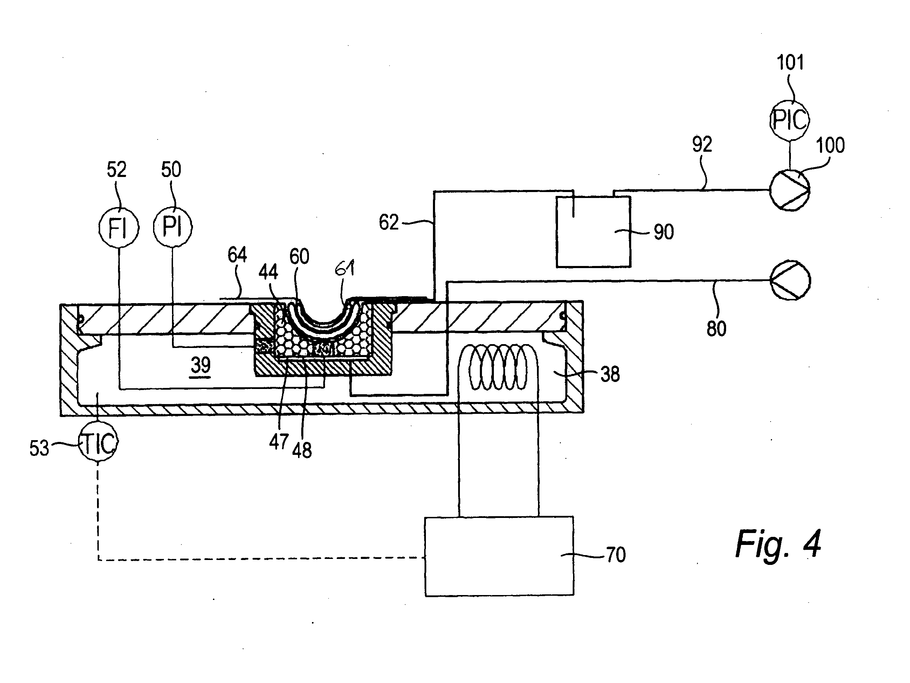

Figur 4 zeigt schließlich eine Ausgestaltung einer Messapparatur im Betrieb mit angeschlossener Unterdruckerzeugungseinheit. Hierbei sind gleiche Bauteile mit gleichen Bezugszeichen bezeichnet. Ebenfalls dargestellt in Figur 4 ist die regelbare Heizung 70 für das Heizbad 39, das im Hohlraum 38 angeordnet ist. Dabei wird zur Temperaturregelung u. a. die Temperatur des Heiz- oder Wasserbades 39 bestimmt, wozu ein Sensor 53 vorgesehen ist.

Darüber hinaus sind, wie zu Figur 2 erläutert, ein Kraftsensor 52 sowie ein Drucksensor 50 vorgesehen, die hier mit den gleichen Bezugszeichen bezeichnet sind.

Über die gesteuerte Wundexsudatzufuhr, die hier mit dem Bezugszeichen 80 versehen ist und der Zuleitung 46 für künstliches Wundexsudat (Fluid) in den vorstehenden Figuren entspricht, wird künstliches Wundexsudat in einen Holraum unterhalb der künstlichen Wunde 44 eingeleitet und dort gleichmäßig über die Oberfläche 47 der künstlichen Wunde mit dem Hohlraum 48 verteilt. Über die Porosität der Glasfritte, die als künstliche Wunde 44 dient, tritt dann Wundexsudat in die Wundkavität 45 ein, in die hier eine mehrlagige Wundauflage 61 des Unterdrucktherapiesystems 60 eingebracht ist. Die Festlegung der Wundauflage 61 an der künstlichen Wunde erfolgt wiederum durch eine Abdeckschicht 64, die klebend an der Messapparatur festgelegt ist. Über den Drainageschlauch 62 erfolgt das Anlegen des Unterdrucks an die Wundauflage 61, wobei der Drainageschlauch 62 über einen Auffangbehälter 90 für Wundexsudat geleitet ist, in dem abgesaugtes Wundexsudat gesammelt werden kann. Der Drainageschlauch ist an einer Unterdruckerzeugungseinrichtung 100, die hier durch eine geregelte Unterdruckpumpe gebildet ist und mit einem Druckdifferenzsensor 101 verbunden ist, festgelegt.

Durch die Anlegung des Unterdrucks wird die Wundauflage 61 in die Wundkavität 45 eingezogen und es wird hierdurch in der Art einer Kompressionstherapie durch den angelegten Unterdruck das Gewebe mit einer Kraft beaufschlagt, die über den Kraftsensor 52 gemessen werden kann. Darüber hinaus kann der angelegte Druck über den Sensor 50 ermittelt werden.

Durch die vorstehend beschriebene Messeinrichtung kann besonders einfach eine künstliche Wunde 44 nachgebildet werden, die insbesondere durch ihre Verschwenkbarkeit verschiedene Situationen, wie beispielsweise einen liegenden Patienten oder einen stehenden Patienten, aber auch Bereiche mit unterschiedlich starker Fluidansammlung, darstellen kann. Über die Verwendung der Glasfritte als künstliche Wunde 44 kann eines besonders gleichmäßige und dabei lebensechte Simulation des Austritts von Wundexsudat aus einer Wunde erzielt werden.

Schließlich kann durch die Verwendung eines Heizbades 39 die künstliche Wunde 44, aber auch die Zuleitung an Wundexsudat 46, 80 temperiert werden, so dass hier Bedingungen, wie sie im Körper eines Menschen oder Tieres herrschen, nachgebildet werden können. Schließlich können über die Vorsehung des Kraftsensors 52 die tatsächlichen Druckverhältnisse an einer Wunde aufgenommen werden.

## Patentansprüche

1. Messapparatur für Unterdrucktherapiesysteme (60) für die Wundbehandlung umfassend eine künstliche Wundeinheit (44), die eine Wandung (43) umfasst, die eine wenigstens einseitig offene, künstliche Wundkavität (45) einschließt, wobei in der Wandung Fluiddurchlässe vorgesehen sind, die über Fluidzuleitungen (46, 80) mit einem Fluid beaufschlagbar sind und wobei die offene Seite der Wundkavität (45) mit einem Unterdrucktherapiesystem (60) abdeckbar ist, wobei über das Unterdrucktherapiesystem (60) an die Wundkavität (45) über eine Unterdruckerzeugungseinrichtung (90, 92, 100) der Messapparatur ein Unterdruck anlegbar ist, **dadurch gekennzeichnet, dass** eine regelbare Heizeinrichtung (39, 70) zur Temperierung der Wundeinheit (44) und/oder des Fluids vorgesehen ist, und die Wandung (43) der künstlichen Wunde (44) durch ein eine offene Porosität aufweisendes Material gebildet ist.

2. Messapparatur nach Anspruch 1, **dadurch gekennzeichnet, dass** die Heizeinrichtung (39, 70) ein Heiz- oder Wasserbad (39) ist.

3. Messapparatur nach Anspruch 1 oder 2, **dadurch gekennzeichnet, dass** die künstliche Wundeinheit(44) an der Messapparatur lösbar und auswechselbar festlegbar ist.

4. Messapparatur nach einem der Ansprüche 1 bis 3, **dadurch gekennzeichnet, dass** in der künstlichen Wundeinheit (44) ein Kraftsensor (52) vorgesehen ist.

5. Messapparatur nach einem der vorangehenden Ansprüche, **dadurch gekennzeichnet, dass** das eine offene Porosität aufweisende Material eine Glasfritte ist.

6. Messapparatur nach einem der vorangehenden Ansprüche, **dadurch gekennzeichnet, dass** die künstliche Wunde (44) an einer Halterung (10, 20) festgelegt und an der Halterung (10, 20) verschwenkbar gelagert ist, wobei die Verschwenkung in verschiedenen Positionen festlegbar ist.

7. Messapparatur nach einem der vorangehenden Ansprüche, **dadurch gekennzeichnet, dass** die Wundkavität (45) mit einer Spüllösungszuleitung verbindbar ist, über die eine Spüllösung in diese einleitbar ist.

## Claims

1. Measurement apparatus for vacuum therapy systems (60) for wound treatment, comprising an artificial wound unit (44) with a wall (43) that encloses a wound cavity (45) open at least on one side, wherein passages for fluid are provided in the wall, which can be supplied with a fluid via fluid delivery lines (46, 80), and wherein the open side of the wound cavity (45) can be covered by a vacuum therapy system (60), wherein the vacuum therapy system (60) can apply a vacuum to the wound cavity (45) via a vacuum generating device (90, 92, 100) of the measurement apparatus, **characterized in that** a controllable heating device (39, 70) is provided for regulating the temperature of the wound unit (44) and/or of the fluid, and the wall (43) of the artificial wound is constituted by material exhibiting open porosity.

2. Measurement apparatus according to the claim 1, **characterized in that** the heating device (39, 70) is a heating bath or a water bath (39).

3. Measurement apparatus according to either one of the claims 1 or 2, **characterized in that** the artificial wound unit (44) can be detachably and replaceably fixed to the measurement apparatus.

4. Measurement apparatus according to any one of the claims 1 to 3, **characterized in that** the artificial wound unit (44) is provided with a force sensor (52).

5. Measurement apparatus according to any one of the previous claims, **characterized in that** the wall (43) of the artificial wound (44) is constituted by a glass frit.

6. Measurement apparatus according to any one of the previous claims, **characterized in that** the artificial wound (44) is fixed on a mount (10, 20) and is held swivel-mounted on the mount (10, 20), wherein the position entered by swiveling can be fixed.

7. Measurement apparatus according to any one of the previous claims, **characterized in that** the wound cavity (45) can be connected to a rinsing solution delivery line, via which a rinsing solution can be introduced into the same.

## Revendications

1. Appareil de mesure pour des systèmes de thérapie par dépression (60) pour le traitement de plaies, comprenant une unité de plaie artificielle (44) qui comprend une paroi (43) laquelle entoure une cavité de plaie artificielle (45) ouverte sur au moins un côté, dans ladite paroi étant prévus des passages de fluide qui peuvent être alimentés en un fluide par des conduites d'alimentation en fluide (46, 80), et le côté ouvert de la cavité de plaie (45) pouvant être recouverte d'un système de thérapie par dépression (60), dans lequel une dépression pouvant être appliquée à la cavité de plaie (45) par ledit système de thérapie par dépression (60) via un dispositif de génération de dépression (90, 92, 100) de l'appareil de mesure, **caractérisé par le fait qu'**un dispositif de chauffage (39, 70) réglable pour la thermorégulation de ladite unité de plaie (44) et/ou du fluide est prévu et que la paroi (43) de la plaie artificielle (44) est formée par une matière présentant une porosité ouverte.

2. Appareil de mesure selon la revendication 1, **caractérisé par le fait que** ledit dispositif de chauffage (39, 70) est un bain de chauffage ou d'eau (39).

3. Appareil de mesure selon la revendication 1 ou 2, **caractérisé par le fait que** ladite unité de plaie artificielle (44) peut être fixée de manière amovible et échangeable sur ledit appareil de mesure.

4. Appareil de mesure selon l'une quelconque des revendications 1 à 3, **caractérisé par le fait qu'**un capteur de force (52) est prévu à l'intérieur de ladite unité de plaie artificielle (44).

5. Appareil de mesure selon l'une quelconque des revendications précédentes, **caractérisé par le fait que** la matière présentant une porosité ouverte est une fritte de verre.

6. Appareil de mesure selon l'une quelconque des revendications précédentes, **caractérisé par le fait que** ladite plaie artificielle (44) est fixée sur un support (10, 20) et est logée à pivotement sur ledit support (10, 20), le pivotement pouvant être bloqué dans des positions différentes.

7. Appareil de mesure selon l'une quelconque des revendications précédentes, **caractérisé par le fait que** ladite cavité de plaie (45) peut être reliée à une conduite d'alimentation en solution de rinçage via laquelle une solution de rinçage peut être introduite dans celle-ci.
